# EUROPEAN PATENT APPLICATION

(11) **EP 1 233 362 A1**
(43) Date of publication of application: **21.08.2002**
(21) Application number: 01310523.4
(22) Date of filing: 17.12.2001
(51) Int. Cl.: G06F 19/00

(54) **Method and apparatus for remote or collaborative control of an imaging system**

(30) Priority: 21.12.2000 US 745320
(71) Applicant: GE Medical Systems Global Technology Company LLC, Waukesha, Wisconsin 53188-1696 (US)
(72) Inventor: Balloni, William J., Menomonee Falls, Wisconsin 53051 (US); Gould, Kristine Louise, Delafield, Wisconsin 53018 (US); Hlaban, Thomas S., Waukesha, Wisconsin 53186 (US); Debbins, Josef Phillip, Waukesha, Wisconsin 53186 (US); Haworth, Robert H., Brookfield, Wisconsin 53005 (US)
(74) Representative: Goode, Ian Roy

(57) **Abstract**

A method and apparatus for remote or collaborative control of an imaging system (40) included in an imaging system environment is disclosed herein. The environment provides an alternate user interface (74, 24, 34) at a location where the remote or collaborative control of the imaging system (40) will occur. The alternate user interface (74, 24, 34) is generated by an application model (72) included in or proximate to the imaging system (40). The application model (72) is in communication with the alternate user interface (74, 24, 34). The alternate user interface (74, 24, 34) may be located remote or local with respect to the imaging system (40).

## Description

The present invention relates generally to an imaging systems environment. More particularly, the present invention relates to an environment configured to permit remote and/or collaborative control of imaging systems provided therein.

Medical imaging systems, such as, magnetic resonance (MR) imaging systems or computed tomography (CT) systems, are presently operated or controlled by a proximately located operator. The operator (e.g., a technician, a physician, other health care provider, etc.) interfaces with a scanner side operator console (located in the same room as the imaging system) or a main operator console (also located proximate to the imaging system but preferably in an adjacent room) to specify parameters relating to and view images resulting from the image acquisition(s) of a subject of interest, such as a patient's particular anatomy, on the imaging system. Presently, the person operating the imaging system (e.g., a technician) may not be the same person interpreting the acquired images (e.g., a radiologist, a service technician). Moreover, the person interpreting or reviewing the images for diagnosis of physiological abnormalities or image system servicing may be remotely located with respect to the imaging system. In such cases, the acquired images are provided on film (or other hard copy medium) or accessed from an image archival server via a communication link, for review at one or more remote locations.

Unfortunately, the person reviewing the acquired images at the remote location may desire alternative images, such as, images with a different scan orientation, image contrast, or surrounding anatomy. The person may also desire to directly operate the imaging system (to specify one or more parameters) to provide a more accurate diagnosis. The person may further desire to monitor the operator of the imaging system during image acquisition to aid or determine the quality of the acquisition parameters and resulting images in real-time.

Thus, there is a need for a method and apparatus that permits local or remote operation of an imaging system. There is also a need for a method and apparatus that permits one or more operators to simultaneously and/or collaboratively control acquisition of and/or view images acquired from an imaging system. There is a further need for a method and apparatus of interfacing with one or more imaging systems during image acquisition or post-image acquisition to perform a variety of system maintenance and support functions. There is a still further need for a method and apparatus that can perform the advantages recited above without sacrificing existing performance or requiring extensive or costly equipment.

One exemplary embodiment of the invention relates to a method for remote or collaborative control of an imaging system. The imaging system is associated with an application model located at a first location. The application model is in communication with the imaging system. The method includes providing a first user interface at the first location, and providing a second user interface at a second location, in response to a request for remote or collaborative control of the imaging system at the second location. The method further includes communicating with the application model via at least one of the first user interface and the second user interface.

Another exemplary embodiment of the invention relates to an apparatus for remote or collaborative control of an imaging system. The imaging system is in communication with a control unit located at a first location. The apparatus includes a second user interface provided at a second location where the remote or collaborative control of the imaging system will occur. The control unit includes a first user interface and an application model. The second user interface is configured to transmit a second command to the control unit and to receive a second user interface update from the control unit. The second user interface is provided in response to a request for remote or collaborative control of the imaging system at the second location.

Still another exemplary embodiment relates to an apparatus for remote or collaborative control of an imaging system. The apparatus includes first means for interfacing at a first location. The apparatus further includes second means for interfacing at a second location, in response to a request for remote or collaborative control of the imaging system at the second location. The apparatus still further includes means for updating located at the first location. The means for updating is configured to receive a second command from the second means for interfacing and transmit a second interface update to the second means for interfacing in response to the second command.

Yet still another exemplary embodiment relates an image generated by the steps comprising providing a first user interface at a first location and a second user interface at a second location, and commanding an imaging system located at a third location with a command from at least one of the first user interface and the second user interface. The image is further generated by the steps of generating an interface update in response to the command to the imaging system, the interface update including data representative of the image. The second user interface is provided at the second location when a remote or collaborative control of the imaging system is requested by a user at the second location.

The preferred embodiment will become more fully understood from the following detailed description, taken in conjunction with the accompanying drawings, wherein like reference numerals denote like elements, in which:
FIG. 1 is a block diagram of an imaging systems environment which employs an embodiment of the present invention; and
FIG. 2 is a block diagram showing a detailed portion of the imaging systems environment of FIG. 1.

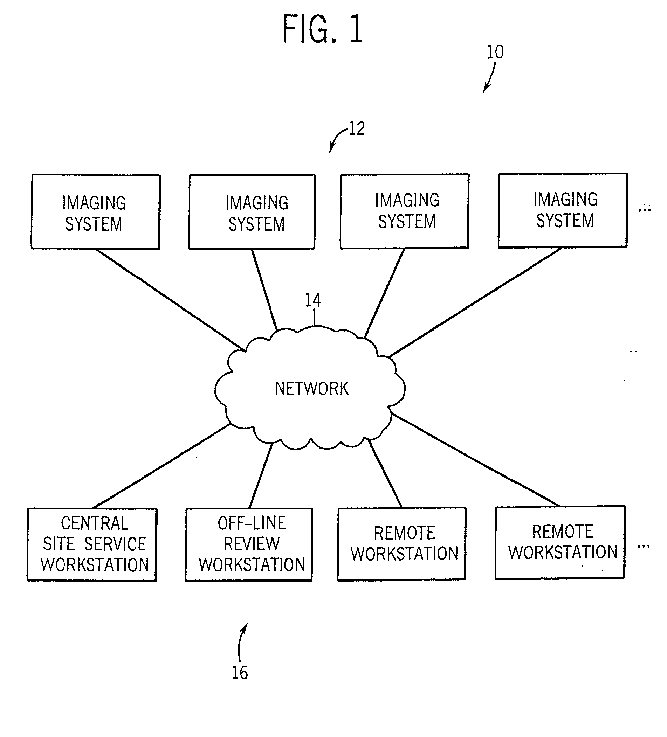

Referring to FIG. 1, there is shown the major components of an imaging systems environment 10. Environment 10 includes imaging systems 12, a communications network 14, and workstations 16. Each of imaging systems 12 and workstations 16 is coupled to communications network 14. Imaging systems 12 include, but are not limited to, magnetic resonance (MR) imaging systems, computerized tomography (CT) imaging systems, nuclear medicine (NM) imaging systems, x-ray systems, and a variety of other imaging systems. It is contemplated that imaging systems 12 are not limited to medical imaging systems and may also include scanners or imaging systems for non-medical uses, such as, for security, geological surveys, etc.

Communications network 14 is preferably an ethernet, fiber optic, or other applicable communication connection related to LAN, WAN, or wireless networking and is configured to utilize protocols such as TCP/IP, CORBA, or Java RMI. Each of workstations 16 can be located proximate or distal to any of imaging systems 12 as long as both are able to communicate with each other via, such as, communications network 14. Workstations 16 can include, but are not limited to, a central site service station, an off-line review station, a central site applications station, a remote reading station, an education/training station, and a remote operator control station.

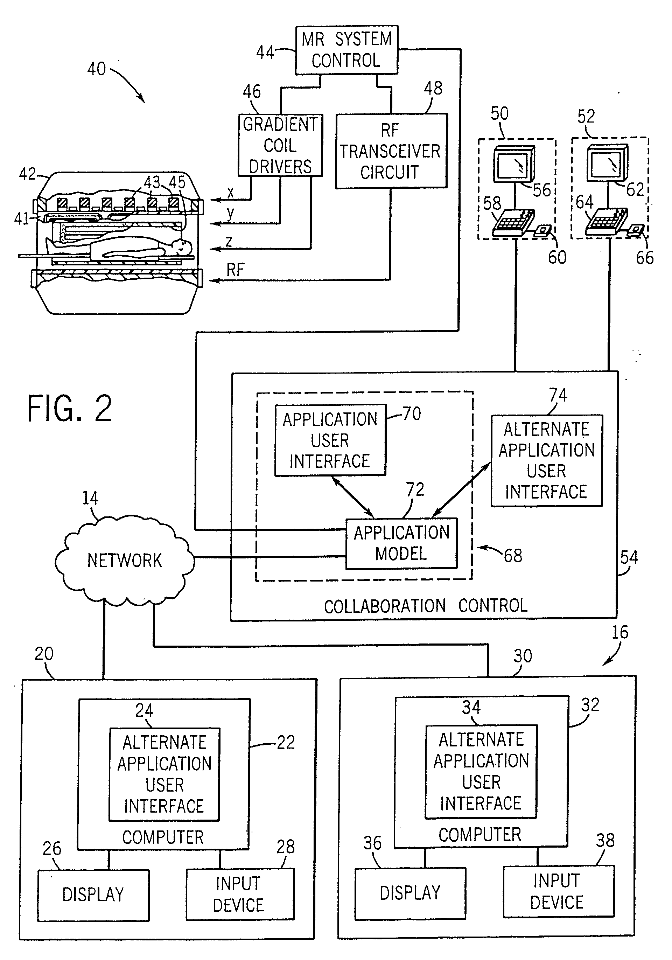

Each of workstations 16 includes a computer (including a memory and a processor), a display, and an input device. The display can include, but is not limited to, a cathode ray tube (CRT) display, a liquid crystal display (LCD), a light emitting diode (LED) display, a plasma display, a touch screen, a projection display, a printer, a plotter, etc. The input device can include, but is not limited to, a mouse, a joystick, a keyboard, a trackball, a touch screen, a light wand, a voice control device, and a custom keyboard/keypad. In FIG. 2, representative workstations 20, 30 are shown. Workstation 20 includes each of a display 26 and an input device 28 coupled to a computer 22. Workstation 30 similarly includes each of a display 36 and an input device 38 coupled to a computer 32. Alternate application user interfaces 24, 34 (to be described in detail hereinafter) are selectively included in computers 22, 32, respectively, and are coupled to communications network 14.

A representative imaging system 40 is also shown in FIG. 2. Imaging system 40 is preferably an MR imaging system. However, it should be understood that exemplary embodiments may alternatively include other types of imaging systems, such as CT imaging systems and other medical imaging systems. Thus, imaging system 40 shown as an MR imaging system is for illustration purposes only and in no way limits the implementation of the exemplary embodiments using other types of imaging systems. Imaging system 40 includes a magnet assembly 42, an MR system control 44, gradient coil drivers 46, a radio frequency (RF) transceiver circuit 48, a magnet side operator console 50, a main operator console 52, and a collaboration control 54.

MR system control 44 couples to gradient coil drivers 46 and RF transceiver circuit 48. Gradient coil drivers 46 (also referred to as gradient amplifiers) and RF transceiver circuit 48 couples to gradient coils and an RF coil, respectively, included in magnet assembly 42. Magnet side operator console 50 and main operator console 52 couple to collaboration control 54. Collaboration control 54 couples to each of communications network 14 and MR system control 44.

Magnet assembly 42 includes gradient coils 41, a main or polarizing magnet 43, and an RF coil 45. Magnet assembly 42 is shown as a closed magnet structure, but may alternatively be an open magnet structure. MR system control 44 preferably includes a set of modules connected together by a backplane including a CPU module, a pulse generator module, a memory module, and an array processor module (not shown). MR system control 44 receives commands from an operator (via collaboration control 54) regarding scan parameters and sequences to be performed. MR system control 44 configures and outputs various signals (including pulse sequence data specifying the timing, length, strength, and shape of the pulses) for the remaining system components to carry out the desired scan sequence. MR system control 44 also receives sensor data and acquired image data from magnet assembly 42 and circuit 48 for processing (such as image data reconstruction), storage, and transmission to the operator.

Gradient coil drivers 46 output signals (e.g., x, y, and z direction signals), to excite corresponding gradient coils 41 included in magnet assembly 42, to produce magnet field gradients for spatially encoding the MR echo signals. RF transceiver circuit 48 outputs pulses to excite RF coil 45 included in magnet assembly 42 and receives MR echo signals picked up by RF coil 45.

Operator console 50 includes a display 56 coupled to a control panel 58, and an input device 60 coupled to control panel 58. Operator console 52 includes a display 62 coupled to a control panel 64, and an input device 66 coupled to control panel 64. Each of displays 56, 62 can include, but is not limited to, a CRT display, an LCD, an LED display, a plasma display, a touch screen, a projection display, a printer, a plotter, etc. Each of input devices 60, 66 is selected from a group including, but not limited to, a mouse, a joystick, a trackball, a touch screen, a light wand, a voice control device, and a custom keyboard/keypad. Each of control panels 58, 64 includes dedicated buttons, knobs, switches, slider indicators, LED indicators, etc., to provide additional interactive functionality.

Operator consoles 50, 52 (also referred to as operator interfaces) are configured to enable the operator to control the production and visualization of images. Conventionally, operator console 50 is located proximate to magnet assembly 42. Operator console 50 is also referred to as a table side or scanner side operator console. Operator console 52 is also proximate magnet assembly 42 and is located outside of the scan room. As such, the operator avoids introducing objects into the scan room during image acquisition (e.g., metallic objects which may damage magnet assembly 42). The operator, who may operate imaging system 40 for long periods of time, also avoids exposure to radiation (whether ionizing (CT) or non-ionizing (MR)) repeatedly emitted from imaging system 40.

Collaboration control 54 is configured to permit one or more operators, local or remote, to interface with imaging system 40. Collaboration control 54 is further configured to permit real-time collaborative control from more than one operator console or workstation. Collaboration control 54 is still further configured to display the interfacing actions and images in real-time in all of the involved operator consoles and/or workstations. Thus, collaboration control 54 may provide a real-time user interface to each operator console or workstation connected to network 14 and which is desirous of controlling, viewing images, and/or otherwise being involved with activities relating to imaging system 40.

In one embodiment, collaboration control 54 includes an application 68 comprised of at least an application user interface 70 and an application model 72. Application user interface 70 and application model 72 are preferably software. Alternatively, application user interface 70 and/or application model 72 may be firmware, hardware, software, and/or combinations thereof (such as an application specific integrated circuit (ASIC)). Collaboration control 54 preferably includes a processor and a memory with corresponding software.

Collaboration control 54 is in communication with MR system control 44 via application model 72 and an application server (not shown) included in MR system control 44. However, it is contemplated that the functionality of MR system control 44 and collaboration control 54 may be embodied in a single component. It is also contemplated that some of the functionality of MR system control 44 or collaboration control 54 may be performed in control 54 or control 44, respectively. Thus, MR system control 44 and collaboration control 54, alone or in combination, perform, among others, data acquisition, waveform or pulse sequence configuration, reconstruction, image presentation, human interface processing, and coordination of such interfacing activities when more than one operator console or workstation are being accessed by users.

Each operator at an operator console or workstation interacts with a given imaging system via an application user interface, application model 72, and network 14. The application user interface may be application user interface 70 (also referred to as the primary application user interface) in collaboration control 54 or an alternate application user interface (also referred to as the non-primary or secondary application user interface) (to be described in detail hereinafter). Preferably, application user interface 70 and application model 72 are in communication with each other and are open or accessible at all times for the lifetime of the application when environment 10 is operational.

When a local operator console (i.e., magnet side operator console 50 or main operator console 52) is accessed by an operator or user (e.g., a technologist, a physician, a service/maintenance provider, etc.), communication with imaging system 40 is provided via application 68 on collaboration control 54. Collaboration control 54 provides application user interface 70 to that local operator console. For example, application user interface 70 includes a graphical user interface (GUI) or other control or viewing mechanisms for the operator to interact with system 40. Through interface 70, the operator can specify an imaging or scan plane, specify the desired image contrast, initiate a scan, request display of stored images, etc.

Commands made to interface 70 are communicated to application model 72. Application model 72 processes these commands and, in turn, communicates with MR system control 44 to complete the requested commands. Completed actions and data from imaging system 40 are transmitted to application model 72 via MR system control 44. Application model 72 may process such information to configure it into an appropriate update to application user interface 70. Application model 72 then transmits a user interface update to interface 70. In this manner, the operator at the local operator console will see the results of his/her request on display 56 and/or control panel 58.

Application model 72 translates user interface commands into actions and calculations, and also receives results of a given scan or scanning session for presentation. As such, application model 72 is involved in, but not limited to, scanner set up (e.g., image contrast, pulse sequence timing, hardware settings, etc.); scanner control; real-time scanner control (e.g., real-time change(s) and/or prescription of image contrast, pulse sequence timing, hardware settings, etc.); timely presentation of one or more images; archiving; networking; and image presentation control for non-electronic formats, such as in film.

When both magnet side operator console 50 and main operator console 52 are accessed, collaboration control 54 is configured to provide application user interface 70 to main operator console 52 and an alternate application user interface 74 to magnet side operator console 50. Application model 72 generates alternate application user interface 74 in response to the second local operator console being initially accessed while the first local operator console is already in use. Alternatively, application user interface 70 may remain with the first local operator console accessed (e.g., magnet side operator console 50) and the alternate interface 74 may be provided to the second operator console accessed (e.g., main operator console 52).

Each of alternate application user interfaces 24, 34, 74 is similar to application user interface 70 and includes substantially the same functionality thereto. Each of alternate interfaces 24, 34, 74 is preferably an identical copy of all or a portion of application user interface 70, such that more than one person may simultaneously drive application model 72. In another embodiment, each of alternate interfaces 24, 34, 74 may be a different interface from application user interface 70 but which is still configured to drive application model 72. Similar to application user interface 70, alternate interface 74 also communicates with application model 72 to transmit commands from an operator to MR system control 44 and to receive operator interface updates in response to the executed commands.

When a person desires to interact with or access information associated with imaging system 40 from at least one remote operator console (i.e., any operator console or workstation that communicates with application model 72 via network 14, such as workstations 16 or workstations 20, 30), a local user interface included in that remote operator console communicates with application model 72 via network 14. In response, application model 72 generates an alternate application user interface to be provided to that remote operator console. Accordingly, the person at this remote operator console can transmit commands to application model 72 via the alternate interface and network 14, and receive user interface updates from application model 72 via network 14 and the alternate interface.

For example, a person on workstation 20 initiates a connection through a local user interface (not shown) included in computer 22. The connection request is transmitted to application model 72 via network 14. Application model 72 generates an alternate application user interface 24 (alternate interface 24 having similar characteristics to alternate interface 74) to be provided to computer 22. Then the person can drive application model 72 via alternate interface 24 and network 14, thereby specifying commands to application model 72 and receiving user interface updates from application model 72 in response to these commands.

A local user interface is preferably included in each remote operator console to initiate connection to imaging system 40 or collaboration control 54, or to permit local access of features and/or data located at a given remote operator console (e.g., reviewing images already stored in a given remote operator console). Workstation 30 and its alternate application user interface 34 are similar to workstation 20 and alternate interface 24, respectively, discussed above. However, it should be understood that each of alternate interface 24, alternate interface 34, or alternate interface 74 would only be generated as needed (i.e., when a person at the corresponding operator console or workstation requests a connection to an imaging system or otherwise wishes to communicate with another operator console or workstation). For example, if main operator console 52 and workstation 20 are accessed, then alternate interface 24 would be generated (such that application user interface 70 and/or alternate interface 24 can drive application model 72) but alternate interfaces 74 and 34 would not exist.

In this manner, a given imaging system can be simultaneously accessed by one or more persons located at local and/or remote locations. All the persons accessing a given imaging system at a given time may be shown similar, if not identical, information in real-time or quasi real-time via corresponding user interfaces, and each may also have the ability to effect the displayed information for him/herself as well as others. Preferably, commands from each of the active user interfaces are processed by the application model, and the application model transmits corresponding user interface updates to all of the active user interfaces. Real-time or quasi real-time refers to continuous monitoring, execution, and updating of operator commands and results as rapidly as possible, as constrained by system performance. Several examples illustrating uses of the remote and/or collaborative control scheme are provided below.

For example, a scanner operator at main operator console 52 and a physician at a reading room (typically remotely located with respect to imaging system 40, such as workstation 20) wish to confer about the orientation and location of the next imaging or scan slice(s) of a patient presently positioned within magnet assembly 42. Using application user interface 70 and alternate interface 24, the scanner operator and the physician, respectively, can "share" a graphical prescription tool to interactively collaborate on the orientation and location of the next imaging slice(s) in real-time. The information displayed on displays 62 and 26 would be the same, such that each would see prescriptions made by the other; and control panel 64, input device 66, or input device 28 would be utilized by the scanner operator or physician, respectively.

In another example, the scanner operator at main operator console 52 may set up a real-time scan (e.g., specify initial parameters and properly position the patient) of the patient positioned within magnet assembly 42. Then the scanner operator can request the physician in a remote reading room (e.g., workstation 20) to operate (e.g., initiate and henceforth control) imaging system 40. This permits the physician to control the rest of the scan session (e.g., resolution of images, length of scan time, scan slice orientation, etc.) without being physically present at either operator console 50 or 52. This and the previous example are also applicable when one or more mobile scanners collaborating with a central facility of physicians or diagnosticians are used in the event of a natural disaster, in a battlefield, a sporting event, etc.

In still another example, training, servicing, troubleshooting, performance evaluation, and/or design evaluation may be carried out with the remote and/or collaborative control scheme. A person (e.g., a central site service engineer) at a central site service workstation can remotely monitor the actions of a scanner operator at the local operator console or at any of workstation 16. Based on this monitoring, the person may provide the scanner operator with instructions via telephone and/or an alternate application user interface regarding correct operation of that imaging system. Similarly, training of the scanner operator(s) may be provided via remote monitoring and collaboration. Moreover, the scanner operators may be evaluated on their performance of specific tasks by a manager or a system designer (e.g., length of time to set up a scan; number of prescription modifications, etc.) to provide job performance data or next generation design data, respectively. Alternatively, when the scanner operator is at the local operator console (so is proximate to a magnet assembly), the engineer may troubleshoot problems associated with that imaging system. The engineer may remotely monitor the imaging system's outputs (relative to the scanner operator and/or engineer's inputs) and request the scanner operator to perform equipment changes or configurations (e.g., placing various test objects within the magnet assembly) to determine the problem and possibly even the solution.

In still yet another example, any of the imaging systems 12 or workstations 16 may be accessed for off-line review of its performance and activities by an off-line review workstation. Such off-line review is preferably performed after the remote and/or collaborative session with a given imaging system has been completed. The off-line review facilitates, among others, maintenance based on actual usage and simultaneous software upgrades.

It should be understood that these and other uses for the scheme are possible. The above discussion and examples in no way limit the scope of the scheme. Remote and/or collaborative control of more than one imaging system may occur at any given time in environment 10, each such imaging system being controlled as described above. The exemplary embodiments of the scheme permits application user interface 70 and an alternate application user interface (e.g., alternate interfaces 74, 24, or 34) to differ, such as when application user interface 70 is configured for a lower resolution display than the display associated with an alternate interface. The scheme permits an operator console or a workstation to connect to application model 72 (and hence be provided with an alternate interface for remote and/or collaborative control) before or during a scan, or while a remote and/or collaborative session is in progress with another workstation.

In this manner, environment 10 enables an application user interface associated with a given imaging system to be replicated in part or in whole, remoted, and supported to facilitate remote and/or collaborative control of that imaging system in quasi real-time or real-time. Since it is physicians that predominantly diagnose and/or make clinical findings upon review of images generated from imaging systems (such as imaging system 40), providing remote and/or collaborative control of imaging systems results in images with higher clinical usefulness, reduced scan time, and reduced patient discomfort and exposure to image-producing radiation (since the scanner operator need no longer acquire a plurality of images to anticipate the types of images the physician may require). Furthermore, providing remote and/or collaborative control makes more efficient use of equipment, personnel, and expertise. Still further, patients need not return for subsequent scan sessions to "correct" imaging deficiencies or oversights from a previous scan session since an expert and/or the person who will ultimately interpret the images will be present to control the images required therefrom.

While the embodiments and application of the invention illustrated in the figures and described above are presently preferred, it should be understood that these embodiments are offered by way of example only. For example, application user interface 70 may communicate with application model 72 via a network or local connection. In another example, a given operator console or workstation may permit remote and/or collaborative control of more than one imaging system. Such an operator console or workstation could include a corresponding number of different user interfaces (each user interface in communication with the application model of a given imaging system) to interact with the plurality of imaging systems.

For completeness, various aspects of the invention are set out in the following numbered clauses:
1. A method for remote or collaborative control of an imaging system (40), the imaging system (40) associated with an application model (72) located at a first location and the application model (72) being in communication with the imaging system (40), the method comprising the steps of:
   providing a first user interface (70) at the first location;
   providing a second user interface (74, 24, 34) at a second location, in response to a request for remote or collaborative control of the imaging system (40) at the second location; and
   communicating with the application model (72) via at least one of the first user interface (70) and the second user interface (74, 24, 34).
2. The method of clause 1, wherein providing a second user interface (74, 24, 34) includes generating the second user interface (74, 24, 34) from the application model (72).
3. The method of clause 2, wherein providing a second user interface (74, 24, 34) includes replicating at least a part of the first user interface (70) using the application model (72) to the second location.
4. The method of clause 1, further comprising commanding the imaging system (40) using at least one of the first (70) and the second (74, 24, 34) user interfaces.
5. The method of clause 4, further comprising updating the first (70) and the second (74, 24, 34) user interfaces in response to at least one command made to the imaging system (40) by at least one of the first (70) and the second (74, 24, 34) user interfaces or in response to at least one response returned from the imaging system (40).
6. The method of clause 5, wherein updating the first (70) and the second (74, 24, 34) user interfaces include the application model (72) generating an interface update in response to the at least one command from the first (70) or the second (74, 24, 34) user interface or in response to the at least one response from the imaging system (40).
7. The method of clause 1, wherein the first location is proximate to the imaging system (40).
8. The method of clause 1, wherein the second location is remote from the first location and the imaging system (40).
9. The method of clause 8, wherein communicating with the application model (72) by the second user interface (74, 24, 34) includes communicating with a communications network (14) coupled between the application model (72) and the second user interface (74, 24, 34).
10. The method of clause 9, wherein the communications network (14) is selected from a group including an intranet, the Internet, a local area network (LAN), a broadband network, a wireless network, and a variety of other networks.
11. The method of clause 1, wherein the second user interface (74, 24, 34) is proximate to the imaging system (40).
12. The method of clause 1, wherein the second location is the first location.
13. The method of clause 12, wherein communicating with the application model (72) includes the first (70) and the second (74, 24, 34) user interfaces directly communicating with the application model (72).
14. The method of clause 12, wherein the first user interface (70), the second user interface (74, 24, 34), and the application model (72) are included in a collaboration control unit (54).
15. The method of clause 1, further comprising providing a third user interface (74, 24, 34) at a third another location where the remote or collaborative control will occur, wherein the locations of the first (70), the second (74, 24, 34), and the third (74, 24, 34) user interfaces are different from each other.
16. The method of clause 1, wherein the first user interface (70) is a user interface selected from a group including a user interface similar to at least a portion of the second user interface (74, 24, 34), and a user interface different from the second user interface (74, 24, 34).
17. An apparatus for remote or collaborative control of an imaging system (40), the imaging system (40) being in communication with a control unit (54) located at a first location, the apparatus comprising a second user interface (74, 24, 34) provided at a second location where the remote or collaborative control of the imaging system (40) will occur, wherein the control unit (54) includes a first user interface (70) and an application model (72), and the second user interface (74, 24, 34) is configured to transmit a second command to the control unit (54) and to receive a second user interface update from the control unit (54), and the second user interface (74, 24, 34) being provided in response to a request for remote or collaborative control of the imaging system (40) at the second location.
18. The apparatus of clause 17, wherein the second user interface (74, 24, 34) is generated from the application model (72) when remote or collaborative control of the imaging system (40) is requested by an operator.
19. The apparatus of clause 17, wherein the second user interface (74, 24, 34) is configured to transmit the second command to the application model (72) and to receive the second user interface update from the application model (72).
20. The apparatus of clause 17, wherein the first user interface (70) is configured to transmit a first command to the application model (72) and to receive a first user interface update from the application model (72).
21. The apparatus of clause 20, wherein the imaging system (40) is controlled via at least one of the first and the second commands from the first (70) and the second (74, 24, 34) user interfaces, respectively.
22. The apparatus of clause 20, wherein the first and the second user interface updates are generated by the application model (72) in response to any of the first command, the second command, or at least one response returned from the imaging system (40).
23. The apparatus of clause 22, wherein the first and the second user interface updates are similar to each other.
24. The apparatus of clause 17, wherein the second location is remote from the imaging system (40) and the first location.
25. The apparatus of clause 24, further comprising a communications network (14) coupled between the application model (72) and the second user interface (74, 24, 34).
26. The apparatus of clause 25, wherein the communications network (14) is selected from a group including an intranet, the Internet, a local area network (LAN), a broadband network, and a wireless network.
27. The apparatus of clause 17, wherein the second location is proximate to the first location.
28. The apparatus of clause 27, wherein the second user interface (74, 24, 34) is included in the control unit (54).
29. The apparatus of clause 17, further comprising a third user interface (74, 24, 34) at a third location where the remote or collaborative control will occur, wherein the locations of the first (70), the second (74, 24, 34), and the third (74, 24, 34) user interfaces are different from each other.
30. The apparatus of clause 17, wherein the second user interface (74, 24, 34) is included in at least one of a local operator console (50, 52) and a remote workstation (20, 30).
31. An apparatus for remote or collaborative control of an imaging system (40), the apparatus comprising:
   first means for interfacing (70) at a first location;
   second means for interfacing (74, 24, 34) at a second location, in response to a request for remote or collaborative control of the imaging system (40) at the second location; and
   means for updating (72) located at the first location and configured to receive a second command from the second means for interfacing (74, 24, 34) and transmit a second interface update to the second means for interfacing (74, 24, 34) in response to the second command.
32. The apparatus of clause 31, wherein the means for updating (72) is further configured to receive a first command from the first means for interfacing (70) and transmit a first interface update to the first means for interfacing (70) in response to the first command.
33. The apparatus of clause 32, wherein the first interface update is transmitted to the first (70) and second (74, 24, 34) means for interfacing in response to the first command, and the second interface update is transmitted to the first (70) and second (74, 24, 34) means for interfacing in response to the second command.
34. The apparatus of clause 31, wherein the second means for interfacing (74, 24, 34) is generated from the means for updating (72) in response to the request for remote or collaborative control from an operator located at the second location.
35. The apparatus of clause 31, wherein the second location is remote from the first location.
36. The apparatus of clause 35, further comprising means for communicating (14) configured to provide communication between the means for updating (72) and the second means for interfacing (74, 24, 34).
37. The apparatus of clause 36, wherein the means for communicating (14) is selected from a group including an intranet, the Internet, a local area network (LAN), a broadband network, and a wireless network.
38. The apparatus of clause 31, wherein the means for updating (72) and the first means for interfacing (70) are located proximate to the imaging system (40).
39. The apparatus of clause 31, wherein the second location is the first location.
40. The apparatus of clause 39, wherein the means for updating (72), the first means for interfacing (70), and the second means for interfacing (74, 24, 34) are included in a collaboration control (54).
41. The apparatus of clause 31, further comprising third means for interfacing (74, 24, 34) at an another location where remote or collaborative control of the imaging system (40) is requested, wherein the first (70), the second (74, 24, 34), and the third (74, 24, 34) means for interfacing are provided at different locations.
42. The apparatus of clause 41, wherein the third means for interfacing (74, 24, 34) is generated from the means for updating (72) in response to a request for remote or collaborative control from an operator located at the another location.
43. The apparatus of clause 41, wherein the means for updating (72) is further configured to receive a third command from the third means for interfacing (74, 24, 34) and transmit a third interface update to the third means for interfacing (74, 24, 34) in response to the third command.
44. The apparatus of clause 43, wherein the first interface update is transmitted to the first (70), second (74, 24, 34), and third (74, 24, 34) means for interfacing in response to the first command, the second interface update is transmitted to the first (70), second (74, 24, 34), and third (74, 24, 34) means for interfacing in response to the second command, and the third interface update is transmitted to the first (70), second (74, 24, 34), and third (74, 24, 34) means for interfacing in response to the third command.
45. The apparatus of clause 31, wherein the imaging system (40) is selected from a group including a magnetic resonance (MR) imaging system, a computerized tomography (CT) imaging system, a nuclear medicine (NM) imaging system, and a x-ray system.
46. An image generated by the steps comprising:
   providing a first user interface (70) at a first location and a second user interface (74, 24, 34) at a second location;
   commanding an imaging system (40) located at a third location with a command from at least one of the first user interface (70) and the second user interface (74, 24, 34); and
   generating an interface update in response to the command to the imaging system (40), the interface update including data representative of the image,
   wherein the second user interface (74, 24, 34) is provided at the second location when a remote or collaborative control of the imaging system (40) is requested by a user at the second location.
47. The image of clause 46, wherein the first location and the second location are remote from each other.
48. The image of clause 47, wherein the third location is the same as the first location or the second location.
49. The image of clause 47, wherein the first, the second, and the third locations are remote from each other.
50. The image of clause 46, wherein the first location and the second location are proximate to each other.
51. The image of clause 50, wherein the third location is the same as the first location or the second location.
52. The image of clause 50, wherein the third location is remote from at least one of the first location and the second location.
53. The image of clause 46, wherein the providing step includes providing the second user interface (74, 24, 34) using an application model (72) in communication with the imaging system (40).
54. The image of clause 46, further comprising communicating to and from the first (70) and the second (74, 24, 34) user interfaces via an application model (72) in communication with the imaging system (40).
55. The image of clause 54, wherein the generating step includes generating the interface update using the application model (72).
56. The image of clause 55, further comprising updating the first (70) and the second (74, 24, 34) user interfaces in response to the interface update.
57. The image of clause 56, wherein the updating step includes displaying the image on a means for displaying (56, 62, 26, 36) associated with each of the first (70) and the second (74, 24, 34) user interfaces.
58. The image of clause 46, wherein the command is selected from a group including image contrast prescription commands, scanning session commands, image acquisition plane prescription commands, archiving commands, pulse sequence prescription commands, image retrieval commands, imaging system configuration commands, and a variety of other commands.

## Claims

1. A method for remote or collaborative control of an imaging system (40), the imaging system (40) associated with an application model (72) located at a first location and the application model (72) being in communication with the imaging system (40), the method comprising the steps of:
providing a first user interface (70) at the first location;
providing a second user interface (74, 24, 34) at a second location, in response to a request for remote or collaborative control of the imaging system (40) at the second location; and
communicating with the application model (72) via at least one of the first user interface (70) and the second user interface (74, 24, 34).

2. An apparatus for remote or collaborative control of an imaging system (40), the imaging system (40) being in communication with a control unit (54) located at a first location, the apparatus comprising a second user interface (74, 24, 34) provided at a second location where the remote or collaborative control of the imaging system (40) will occur, wherein the control unit (54) includes a first user interface (70) and an application model (72), and the second user interface (74, 24, 34) is configured to transmit a second command to the control unit (54) and to receive a second user interface update from the control unit (54), and the second user interface (74, 24, 34) being provided in response to a request for remote or collaborative control of the imaging system (40) at the second location.

3. The apparatus of claim 2, wherein the second user interface (74, 24, 34) is generated from the application model (72) when remote or collaborative control of the imaging system (40) is requested by an operator.

4. The apparatus of claim 2, wherein the second user interface (74, 24, 34) is configured to transmit the second command to the application model (72) and to receive the second user interface update from the application model (72).

5. The apparatus of claim 2, wherein the first user interface (70) is configured to transmit a first command to the application model (72) and to receive a first user interface update from the application model (72).

6. The apparatus of claim 2, further comprising a third user interface (74, 24, 34) at a third location where the remote or collaborative control will occur, wherein the locations of the first (70), the second (74, 24, 34), and the third (74, 24, 34) user interfaces are different from each other.

7. An apparatus for remote or collaborative control of an imaging system (40), the apparatus comprising:
first means for interfacing (70) at a first location;
second means for interfacing (74, 24, 34) at a second location, in response to a request for remote or collaborative control of the imaging system (40) at the second location; and
means for updating (72) located at the first location and configured to receive a second command from the second means for interfacing (74, 24, 34) and transmit a second interface update to the second means for interfacing (74, 24, 34) in response to the second command.

8. The apparatus of claim 7, wherein the means for updating (72) is further configured to receive a first command from the first means for interfacing (70) and transmit a first interface update to the first means for interfacing (70) in response to the first command.

9. The apparatus of claim 7, wherein the second means for interfacing (74, 24, 34) is generated from the means for updating (72) in response to the request for remote or collaborative control from an operator located at the second location.

10. An image generated by the steps comprising:
providing a first user interface (70) at a first location and a second user interface (74, 24, 34) at a second location;
commanding an imaging system (40) located at a third location with a command from at least one of the first user interface (70) and the second user interface (74, 24, 34); and
generating an interface update in response to the command to the imaging system (40), the interface update including data representative of the image,
wherein the second user interface (74, 24, 34) is provided at the second location when a remote or collaborative control of the imaging system (40) is requested by a user at the second location.
